# EUROPEAN PATENT SPECIFICATION

(11) **EP 2 630 986 B1**
(45) Date of publication and mention of the grant of the patent: **10.12.2014**
(21) Application number: 11834615.4
(22) Date of filing: 19.10.2011
(51) Int. Cl.: A61N 1/05, A61N 1/36, A61N 5/06, A61B 1/00, A61B 1/005, A61B 5/00, G02B 23/24

(54) **LIQUID CRYSTAL POLYMER-BASED ELECTRO-OPTRODE NEURAL INTERFACE, AND METHOD FOR PRODUCING SAME**
FLÜSSIGKRISTALLINE POLYMERBASIERTE ELEKTROOPTRODEN-NEURALSCHNITTSTELLE UND HERSTELLUNGSVERFAHREN DAFÜR
INTERFACE NEURONALE ÉLECTRO-OPTRODE À BASE D'UN POLYMÈRE CRISTAL LIQUIDE, ET SON PROCÉDÉ DE PRODUCTION

(30) Priority: 06.09.2011 KR 20110090040; 21.10.2010 KR 20100103026
(43) Date of publication of application: 28.08.2013
(73) Proprietor: M.I. Tech Co., Ltd., Gyeonggi-do 451-864 (KR)
(72) Inventor: PARK, Se-Ik, Pyeongtaek-si Gyeonggi-do 451-864 (KR); CHANG, Jin-Woo, Pyeongtaek-si Gyeonggi-do 451-864 (KR); KIM, Sung June, Pyeongtaek-si Gyeonggi-do 451-864 (KR); LEE, Sung Eun, Pyeongtaek-si Gyeonggi-do 451-864 (KR); LEE, Seung Woo, Pyeongtaek-si Gyeonggi-do 451-864 (KR); KIM, Shin Ae, Pyeongtaek-si Gyeonggi-do 451-864 (KR); MIN, Kyou Sik, Pyeongtaek-si Gyeonggi-do 451-864 (KR); MOON, Hyo Woon, Pyeongtaek-si Gyeonggi-do 451-864 (KR); SHIN, Hyung-Cheul, Pyeongtaek-si Gyeonggi-do 451-864 (KR); KIM, Jin Won, Pyeongtaek-si Gyeonggi-do 451-864 (KR)
(74) Representative: Karl, Frank
(86) International application number: PCT/KR2011/007778
(87) International publication number: WO 2012/053815

(56) References cited:
- WO-A1-93/23495
- WO-A1-2010/040142
- WO-A2-03/071223
- GB-A- 2 304 723
- US-A1- 2008 033 502
- US-A1- 2009 088 680
- US-A1- 2009 093 403
- US-A1- 2009 118 800
- US-A1- 2009 156 918
- YOON-KYU SONG ET AL: "A Brain Implantable Microsystem with Hybrid RF/IR Telemetry for Advanced Neuroengineering Applications", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22 August 2007 (2007-08-22), pages 445-448, XP031336198, ISBN: 978-1-4244-0787-3
- S. W. LEE; J. M. SEO; S. HA; E. T. KIM; H. CHUNG; S. J. KIM: "Development of microelectrode arrays for artificial retinal implants using liquid crystal polymers", INVEST OPHTHALMOL VIS SCI, vol. 50, no. 12, December 2009 (2009-12), pages 5859-5866, XP002720908,
- CORBETT ET AL: "Polymer-based microelectrode arrays", MATERIALS RESEARCH SOCIETY SYMPOSIUM PROCEEDINGS, MATERIALS RESEARCH SOCIETY, PITTSBURG, PA; US, vol. 926, 1 January 2006 (2006-01-01), pages 46-51, XP009161793, ISSN: 0272-9172, DOI: 10.1557/PROC-0926-CC06-02 [retrieved on 2011-02-01]

## Description

### Technical Field

The present invention relates to a neural interface which is inserted into a body and a method of manufacturing the same and, more particularly, to a liquid crystal polymer-based electro-optrode neural interface including an electrode and an optrode integrated together, and to a method of manufacturing the same.

### Background Art

As is well known in the art, neural interfaces such as electrodes and optrodes for stimulating deep brain regions or measuring neural signals from deep brain regions are responsible for very important functions in neural prostheses such as cochlear implants, artificial thin-films, deep brain stimulators, etc., or in neuroscience research including biosignal measurement, etc. The neural interfaces are used to stimulate neural tissue of animals, including human beings, or to record neural signals or biosignals generated from neutral tissue. Neural stimulation or neural signal recording is carried out using an electrical process or an optical process.

Electrodes are mainly manufactured using semiconductor materials such as silicon, SOI (Silicon-on-Insulator), etc., or metal materials such as platinum, tungsten, etc., through which potential differences of cells are induced or recorded (see Citation Document [1]).

Optrodes are made using transparent materials through which light passes, and cells may be stimulated with light using techniques such as optogenetics, etc. (see Citation Document [2]).

Also, optrodes may record neural signals of cells in response to light, such as surface Plasmon, changes in transmittance of near infrared rays, etc. (see Citation Documents [3] and [4]).

However, conventional materials for electrodes or optrodes have mechanical properties which deteriorate easily, such as in silicon, or have poor rigidity making them unsuitable for use in a brain and thus requiring additional supports. Hence, such materials are mostly limited in applications to implantable neural interfaces, and methods using supports, etc., should be devised (see Citation Document [5]).

Also, materials for use in implantable techniques such as neural interfaces should be based on biocompatibile materials or should be composed exclusively of biocompatibile materials. In the case where a neural interface is applied to a deep brain region, because there should be no undesirable effects caused by the support in the course of signal transmission via the neural interface, a material for use in manufacturing an optical neural interface has to have rigidity. Furthermore, electrodes currently used in neural prosthetic devices are problematic in terms of very high production costs because a batch process which may decrease the production costs, such as a semiconductor process, is not used.

Because materials for neural interfaces of neural prosthetic devices, to which a batch process may be applied in future, are sufficiently competitive, those having strength adapted to be inserted into a brain, being biocompatible and able to decrease the production costs using a batch process are advantageously required.

However, most conventional techniques concerning electrodes or optrodes have been separately studied and tested. In some research groups, prior research into combining these two techniques to construct neural interfaces in vitro was conducted, but research in vivo has not yet been introduced.

Also, when combinations of optrodes and electrodes are applied to stereotaxic operation, it will be expected that the operation position may be rapidly checked, thus shortening the operation time and reducing effects caused by gliosis due to immune responses and effects of artifact signals by stimulation, but neural interfaces having combined optrodes and electrodes are not structures that can be applied to stereotaxic operation (see Citation Document [8]).

For example, in the case of an electrode insertion operation for DBS (Deep Brain Simulator), it is conventionally conducted in such a manner that the insertion position is checked using MRI scanning and ATLAS (anatomical map of the brain) before the electrode insertion operation, and the operation is carried out using stereotaxic operation equipment.

However, it is difficult to execute the operation because of diverse factors including different brain positions or unique brain structures in each person. To solve such problems, methods wherein neural signals are recorded using MER (Micro Electrode Recording) and then stimulation electrodes are inserted are being mainly adopted. In these methods, a micro electrode for recording a neural signal is inserted using a stereotaxic operation device before electrode insertion for DBS. The neural signals are recorded depending on the inserted position to check the correct location of the electrode, after which the micro electrode for recording a neural signal is removed, and a stimulation electrode is inserted again. This is because, upon puncturing of the skull to form a bur hole, the pressure of the skull changes and the spinal fluid of the brain flows out of the brain, and thus there occurs a shift in which the position of the brain is changed and is different than it would be during MRI scanning.

However this method has problems, as follows. ① The operation time is lengthened, undesirably increasing loss of body fluids including the spinal fluid of the brain, blood, etc. ② Two kinds of electrodes are alternately inserted, undesirably increasing the risk of injury to cerebral blood vessels (the recording electrode has a smaller diameter than the stimulation electrode). ③ The insertion position of the stimulation electrode is adjusted to a different position, instead of the insertion route of the recording electrode, unnecessarily causing damage to the brain tissue.

Unlike this, there are proposed insertion methods without MER using MRI information and ATLAS (anatomical map of the brain). Methods using anatomical map information of the brain are used to determine the final insertion position via approximate coordinate systems based on MRI or C-arm, thus shortening the operation time to thereby reduce the loss of body fluids, but it is very difficult to exactly position the stimulation electrode due to minor shifting of the brain during the operation.

When a specific external object is implanted in vivo, there occurs gliosis in which the brain tissue encloses the electrode recognized as an impurity due to the immune response. In this case, transmission of a stimulus electrical signal to the tissue to be stimulated by the electrode may become problematic, thus reducing stimulation effects and therapeutic effects. Hence, in order to provide appropriate stimulation effects, there may occur a situation in which the electrode insertion operation should be carried out again at an alternative position, undesirably increasing a burden on a patient. In the case of the electrode inserted into the brain, all of a series of procedures, from MRI scanning to the insertion operation, should be undesirably conducted again.

Finally in the case where electrical stimulation is applied to cells and the neural signal is recorded with the electrode, the stimulus signal is directly recorded, separately from the neural signal in response to the cell stimulation, which is called an artifact signal. This signal is formed immediately after the stimulation and has a magnitude greater than that of the neural signal. To show the response immediately after the stimulation, an additional process for removing the artifact signal is required.

As mentioned above, the neural interface device to be inserted in vivo should be based on a biocompatible material and should have strength adapted to be inserted into a brain. Also, this interface should be able to be inexpensively manufactured using a batch process from the point of view that it would have a great influence on the popularization and market expansion of neural prosthetic devices. In the case of the electrode used in the neural interface device, an artifact signal in response to the electrical stimulation is recorded, and thus problems due to the need for an additional process for actually separating the neural signal and an additional reference electrode, and due to reduced effects owing to generation of gliosis, should be solved. Moreover, rapid operations should become possible, compared to conventional stereotaxic operations which require a long operation time because of MRI or CT scanning during the operation.

The present inventors have found that a liquid crystal polymer has strength adapted to be inserted into a brain, is biocompatible, and may be subjected to a batch process thus reducing the production cost (see Citation Documents [6] and [7]), and the stereotaxic operation may be conducted using the combined optrode and electrode and thus the operation position may be rapidly checked thereby reducing the operation time, effects due to gliosis may be reduced by the optrode as light may pass through cells to some extent (see Citation Document [9]), and also the case where the neural signal is detected with light is not affected by the artifact signal, thus simplifying the post-treatment procedure (see Citation Documents [3] and [4]).

### [Citation List]

### [Patent Literature]

[1] US Patent No. 6644552 (Registration date: November 04, 2003)
[2] Korean Unexamined Patent Application No. 2010-0081862 (Laid-open date: July 15, 2010)
[3] Korean Unexamined Patent Application No. 2010-0056876 (Laid-open date: May 28, 2010)
[4] Korean Unexamined Patent Application No. 2010-0056872 (Laid-open date: May 28, 2010)
[5] Korean Unexamined Patent Application No. 2010-0010714 (Laid-open date: February 02, 2010)

### [Non-Patent Literature]

[1] "A High-Yield Fabrication Process for Silicon Neural Probes", Seung Jae Oh, Jong Keun Song, Jin Won Kim, and Sung June Kim, IEEE Transactions on Biomedical Engineering, Vol. 53, no. 2, pp. 351-354, Feb. 2006.
[2] "Next-generation optical technologies for illuminating genetically targeted brain circuits". Deisseroth K, Feng G, Majewska AK, Miesenbock G, Ting A, Schnitzer MJ, J. Neurosci. 26 (41): 10380-10386, October 2006.
[3] "Spectrum measurement of fast optical signal of neural activity in brain tissue and its theoretical origin", Jonghwan Lee and Sung June Kim, NeuroImage, Vol. 51, no. 2, pp. 713-722, 2010.
[4] "Optical Measurement of Neural Activity Using Surface Plasmon Resonance", Shin Ae Kim, Kyung Min Byun, Jonghwan Lee, Jung Hoon Kim, Dong-Ghi Albert Kim, Hyoungwon Baac, Michael L. Shuler, and Sung June Kim, Optics Letters, Vol. 33, no. 9, pp. 914-916, May. 2008.
[5] "Polyimide based neural implants with stiffness improvement", Keekeun Lee, Amarjit Singh , Jiping He, Stephen Massia , Bruce Kim, Gregory Raupp, Sensors and Actuators B , Vol. 102,Issue 1, pp.67-72, Sep. 2004
[6]"Development of microelectrode arrays for artificial retinal implants using liquid crystal polymers", S. W. Lee, J. M. Seo, S. Ha, E. T. Kim, H. Chung, and S. J. Kim, Invest Ophthalmol Vis Sci, Vol. 50, no. 12, pp. 5859-5866, Dec. 2009.
[7] M.S. Thesis, "A Study on a LCP-based Depth-type Neural Probe", S. E. Lee, Department of electrical engineering and computer science, College of Engineering, Seoul National University, Seoul, Korea, 2010.
[8]"A 16-site neural probe integrated with a waveguide for optical stimulation", I.-J. Cho, H. W. Baac, and E. Yoon, in Proc. MEMS 2010 Conference, Hong Kong, China, January 24-28, 2010, pp. 995-998.
[9]Optical Absorption of Untreated and Laser-irradiated Tissues, D.K. SARDAR, B.M. ZAPATA, C.H. HOWARD, Lasers in Medical Science 1993, 8:205-209
[10] Polymer-Based Microelectrode Arrays, Scott Corbertt, Joe Ketterl, and Tim Johnson, Master, Res.Soc.Symp. Proc, Vol. 926
[11] Novel Biomedical Implant Interconnects Utilizing Micromachined LCP, Robert Dean, Jenny Weller, Mike Bozack, Brian Farrell, Linas Jauniskis, Josept Ting, David Edell, Jamile Hetke, Proc. of SPIE Vol.5515

### Disclosure

### Technical Problem

Accordingly, an object of the present invention is to provide a liquid crystal polymer (LCP)-based electro-optrode neural interface having integrated electrode and optrode and a method of manufacturing the same.

### Technical Solution

### Advantageous Effects

According to embodiments of the present invention, design and fabrication techniques of electrodes for stimulating deep brain regions, including the combination of optical technology and electrical and electronic technology, enable electrode design in which an optical technique for recording a neural signal without an artifact signal and an electrode technique for electrically recording a neural signal are embodied on a single electrode. Further, the use of LCP (liquid crystal polymer) and a semiconductor process enables the formation of various kinds of electrode site designs and multifunctional electrodes including fluidic channel, etc., and also enables the development for a coupling method with an optical fiber to achieve an optical technique. Furthermore, an integrated electrode can be provided in such a manner that the optical fiber is surrounded with an adhesive sheet in a thermoplastic thin-film form. A variety of neural signal recording methods and stimulation methods can be applied depending on needs, and the neural signal can be more exactly recorded using electrical and optical recording methods. Moreover, continuous disease treatment is possible using electrical stimulation as in conventional electrodes and optical stimulation as in specific cases such as generation of gliosis. MER and stimulation electrode insertion can be completed using one electrode operation, and the operation time can be shortened, thus reducing burdens on patients and operators, and also the electrode position can be monitored in real time, making it possible to perform a more exact electrode insertion operation.

### Description of Drawings

FIG. 1 is a perspective view illustrating an electro-optrode neural interface according to an embodiment of the present invention;
FIG. 2 is a cross-sectional view taken along the line II-II of FIG. 1 illustrating the electro-optrode neural interface;
FIG. 3 is a perspective view illustrating the electrode layer configuration of the electro-optrode neural interface of FIG. 1;
FIGS. 4a to 4g are a flowchart illustrating a process of manufacturing the electro-optrode neural interface of FIG. 1;
FIG. 5 is a flowchart illustrating another process of manufacturing the electro-optrode neural interface of FIG. 1;
FIG. 6 is a perspective view illustrating the entire configuration of an electro-optrode neural interface according to another embodiment of the present invention; and
FIG. 7 is a flowchart illustrating a process of manufacturing the electro-optrode neural interface of FIG. 6.

### Best Mode

Hereinafter, a detailed description will be given of embodiments of the present invention with reference to the appended drawings.

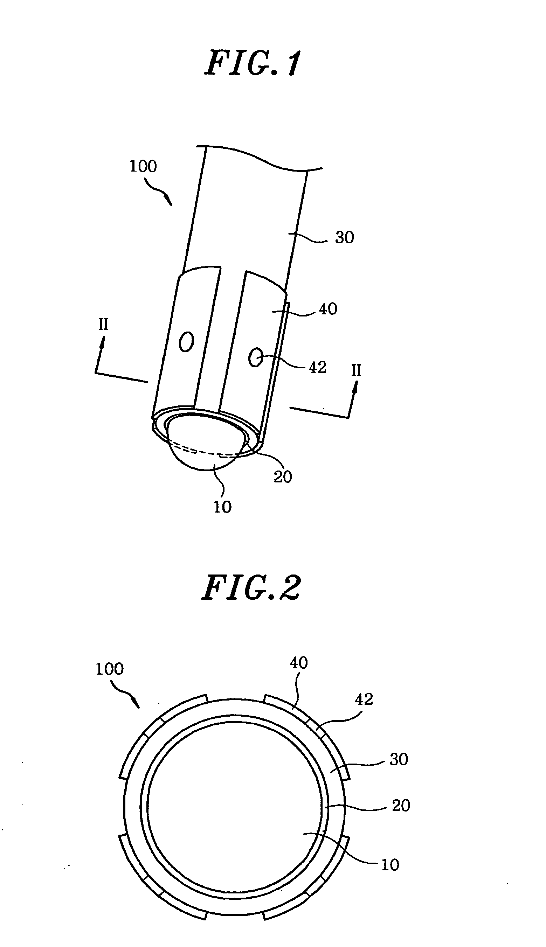

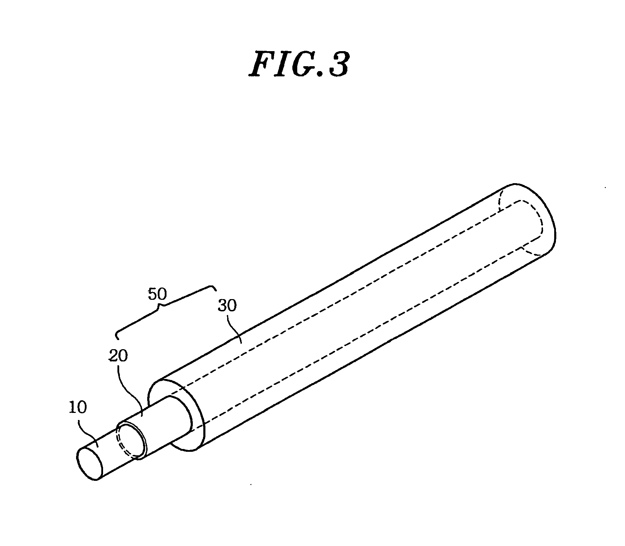

FIG. 1 is a perspective view illustrating an electro-optrode neural interface according to an embodiment of the present invention, FIG. 2 is a cross-sectional view taken along the line II-II of FIG. 1 illustrating the electro-optrode neural interface, and FIG. 3 is a perspective view illustrating the electrode layer configuration of the electro-optrode neural interface of FIG. 1.

As illustrated in FIGS. 1 to 3, the electro-optrode neural interface 100 according to the embodiment of the present invention is connected to an implanted device (not shown), so that biosignals collected in vivo are transferred to the implanted device, and the implanted device executes signal processing, storage and analysis of the transferred biosignals and sends the obtained results to the outside. The electro-optrode neural interface 100 includes an optical fiber 10 which is extended so as to be insertable into the body and is positioned at a core thereof so as to form an optical electrode portion, an adhesive sheet 20 surrounding the optical fiber 10, an LCP electrode layer 30 wound on the optical fiber 10 by means of the adhesive sheet 20 and pre-manufactured using a semiconductor process, and a stimulation electrode site 40 attached to the adhesive sheet 20 at a distal end of the LCP electrode layer 30.

The LCP electrode layer 30 is made of thermoplastic low-temperature LCP or high-temperature LCP, and may form a multi-channel electrode using a semiconductor process or a MEMS process. When the LCP electrode layer 30 is formed with the multi-channel electrode, information about not only one place, but of various places in vivo may be obtained from respective electrodes.

The adhesive sheet 20 functions to adhere or fix the LCP electrode layer 30 to the optical fiber 10, and includes a low-temperature LCP film or polyurethane having adhesiveness and biocompatibility. The adhesive sheet 20 and the LCP electrode layer 30 are coupled to each other to form an LCP sample 50. In the case where the LCP electrode layer 30 is integrated with the adhesive sheet 20 using a semiconductor process, like the LCP sample 50, the LCP electrode layer 30 may be omitted, and thus the diameter of the electro-optrode neural interface may decrease.

The optical fiber 10, which is a part of the optical electrode, may perform optical neural signal measurement or stimulation, and acts as a lens such as an endoscope upon operation so that the interface is inserted into the body while avoiding cerebral blood vessels. Accordingly, a special optical fiber which withstands a temperature of 280 ∼ 290°C, for example, an optical fiber made of polyimide, polyurethane, etc., is preferably used. The end of the optical fiber 10 may have a hemispherical shape as illustrated in FIG. 1, but may have a variety of shapes such as a conical shape, a flat surface, etc., depending on the cutting and processing type.

The stimulation electrode site 40 is made of a metal, for example, Au or Pt, having a thickness of hundreds of nanometers through which an electrical stimulation waveform passes, and executes current/optical signal recording and electrical stimulation. The stimulation electrode site 40 has a fluidic channel 42 configured to inject a drug into the region in which the electrode is inserted.

The electro-optrode neural interface according to the present invention is formed by combining a micro electrode for recording a neural signal and a stimulation electrode, has high long-term stability, and is configured such that a neural electrode is formed using the LCP film 50 comprising the LCP electrode layer and the adhesive sheet 20 integrated with each other, and the optical fiber 10 is surrounded therewith.

The neural electrode using the LCP film 50 comprising the LCP electrode layer and the adhesive sheet 20 integrated with each other plays a role in recording the neural signal and performing multiple functions including electrical stimulation, drug delivery, etc., and the optical fiber 10 is responsible for recording the neural signal and for the function of the stimulation electrode.

As the neural signal recording electrode and the stimulation electrode are integrated with each other, the electrode insertion operation need only be completed once, thus shortening the operation time.

The electro-optrode neural interface according to the present invention is able to use the optical fiber as an endoscope and thus whether the cerebral blood vessel is present or not may be observed with the naked eye in real time using the overall reflective property of the optical fiber. Also, when the integration of peripheral circuits is accomplished, the electro-optrode neural interface according to the present invention enables the direction of electrode entry to be changed variously like in the case of currently used endoscopes, and may thus be developed into a future electrode that is inserted up to the stimulus position while avoiding causing damage to cerebral blood vessels, in lieu of the linear electrode insertion which is currently mainly performed. Ultimately, difficulties in monitoring the position of the electrode are solved.

Although gliosis is not entirely avoided when using external materials, the stimulation electrode of the invention is an optical electrode using an optical technique and thus stimulation of tissue using the optical electrode, namely, optical stimulation, is possible even when gliosis surrounds the electrode, thereby solving problems of gliosis caused by the immune response, etc.

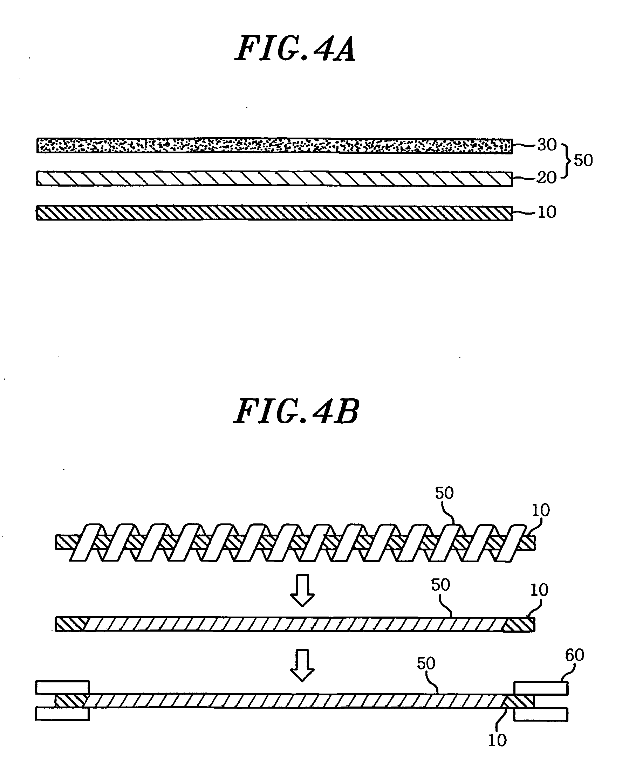

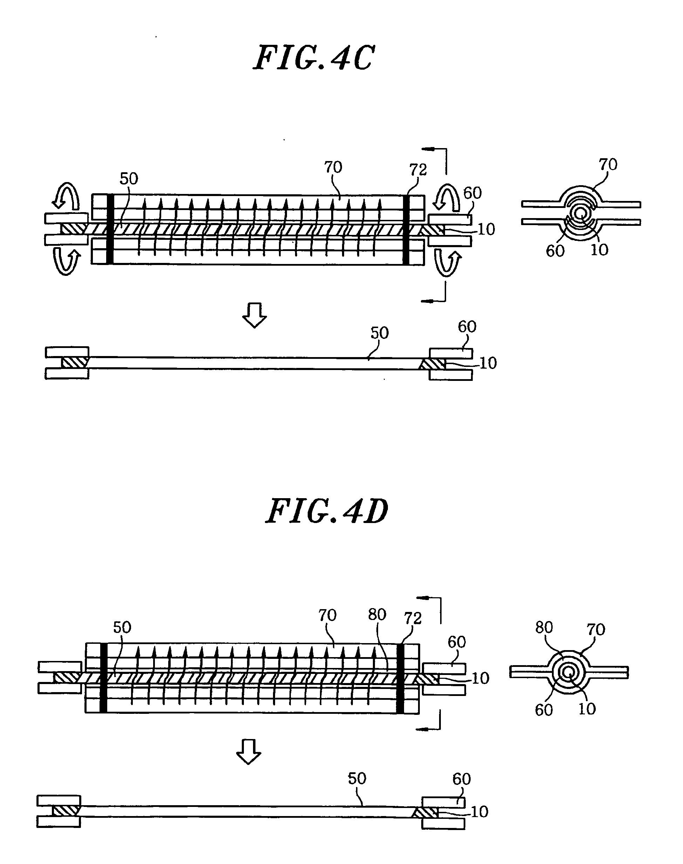

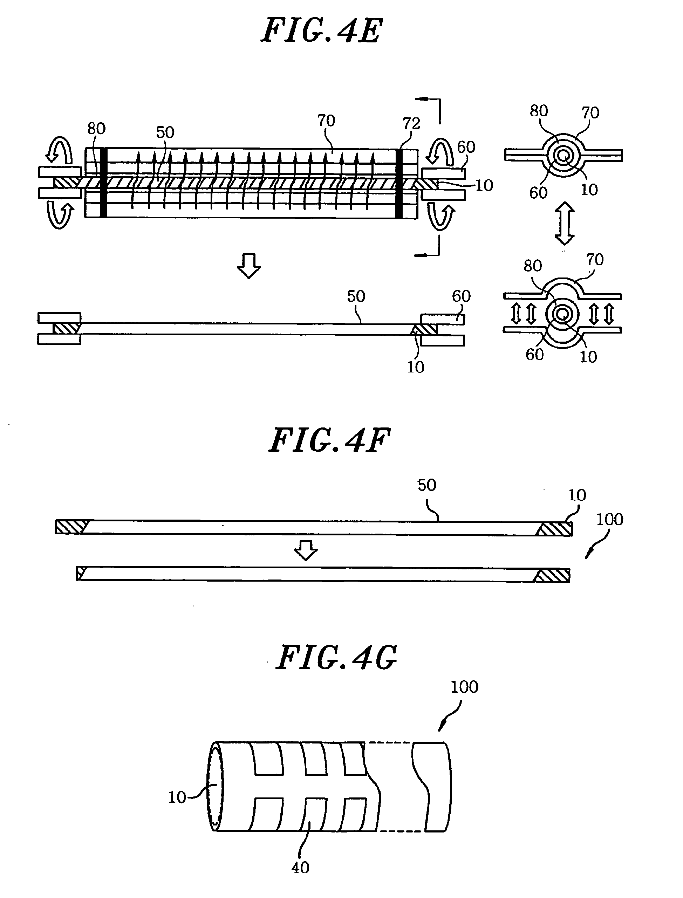

The method of manufacturing the electro-optrode neural interface according to the present invention is described below with reference to FIG. 4.

As illustrated in FIG. 4a, an LCP electrode layer 30, which is pre-manufactured in a long tape form, an adhesive sheet 20, and an optical fiber 10 that withstands a temperature of about 300°C are prepared. The pre-manufactured LCP electrode layer 30 may be integrated with the adhesive sheet 20 using a semiconductor process, and the LCP electrode layer and the adhesive sheet, which are integrated with each other, refer to an LCP sample 50. For the sake of description, the manufacturing method using the LCP sample 50 is described.

Subsequently, as illustrated in FIG. 4b, the LCP sample 50 is densely wound on the optical fiber 10, and the optical fiber 10 is mechanically held to a cylindrical optical fiber fixer 60. As such, the optical fiber 10 is held by being pulled at a predetermined tension so that pressure applied to the LCP sample 50 is maintained constant.

Subsequently, as illustrated in FIG. 4c (the right is a cross-sectional view in the arrow direction), the prepared LCP sample 50 is placed in a metal mold 70 and heated while being rotated. The heating temperature is set so that the temperature applied to the LCP sample 50 is about 275 ∼ 280°C, and the LCP sample 50 is rotated in non-contact type so as not to come into direct contact with the metal mold 70. This is intended to prevent thermal deformation of the optical fiber 10, which is unnecessary and undesirable. In the drawing, the reference numeral 72 is an alignment pin of the LCP 50 and the metal mold 70.

As an alternative heating process, as illustrated in FIG. 4d (the right is a cross-sectional view in the arrow direction), the prepared LCP sample 50 is placed in a metal mold 70 and heated. A repulsive layer 80 made of Teflon is disposed between the optical fiber 10 and the metal mold 70 in order to prevent the electrode layer from being melted and attached to the metal mold 70 during the heating. The heating temperature is set so that the temperature applied to the LCP sample 50 is about 275 ∼ 280°C. This case is performed when heat resistance of the optical fiber 10 is very high, and is advantageous because the process time is short and the roughness of the entire electrode is low.

As another alternative heating process, a mixed heating process including the non-contact type and the contact type may be performed. As illustrated in FIG. 4e (the right is a cross-sectional view in the arrow direction), the mixed heating process is performed by disposing a repulsive layer 80 between the LCP sample 50 and the metal mold 70 and periodically tapping the LCP sample 50 by the metal mold 70 at a regular temporal interval, that is, repeatedly applying predetermined pressure. Furthermore, the LCP sample 50 is heated while being rotated in the metal mold 70. The heating temperature is set so that the temperature applied to the LCP sample 50 is about 275 ∼ 280°C. As the metal mold 70 comes into periodic contact with the LCP sample 50, the thermal deformation of the optical fiber 10, which is unnecessary and undesirable, is prevented.

The above-mentioned heating process enables the LCP electrode layer to be melted by means of heat and pressure so as to attach the adhesive sheet and the optical fiber to each other. This refers to lamination. While the LCP electrode layer is melted under predetermined temperature and pressure conditions, it is coupled to the optical fiber.

After completion of the heating process, as illustrated in FIG. 4f, the tip of the optical fiber 10, which is unnecessarily long, is cut using a fiber cutter, thus manufacturing the electro-optrode neural interface 100 according to the present invention.

Finally, as illustrated in FIG. 4g, a stimulation electrode site 40 is attached to the manufactured electro-optrode neural interface 100. In the electro-optrode neural interface 100, the portion where the optical fiber 10 is exposed is responsible for recording the neural signal using the optical signal, checking for the presence of the cerebral blood vessel upon an electrode insertion operation to execute optical simulation, and measuring the reflectance of the optical signal during the insertion, so that the position of the inserted electro-optrode neural interface may be monitored in real time. Even when gliosis occurs, deep brain stimulation effects may be induced via optical stimulation.

The stimulation electrode site 40 wherein the electrical signal is recorded and the electrical stimulation is executed may have multiple channels depending on the type of design, and impedance or charge injection limit may become diversified depending on the material for the site.

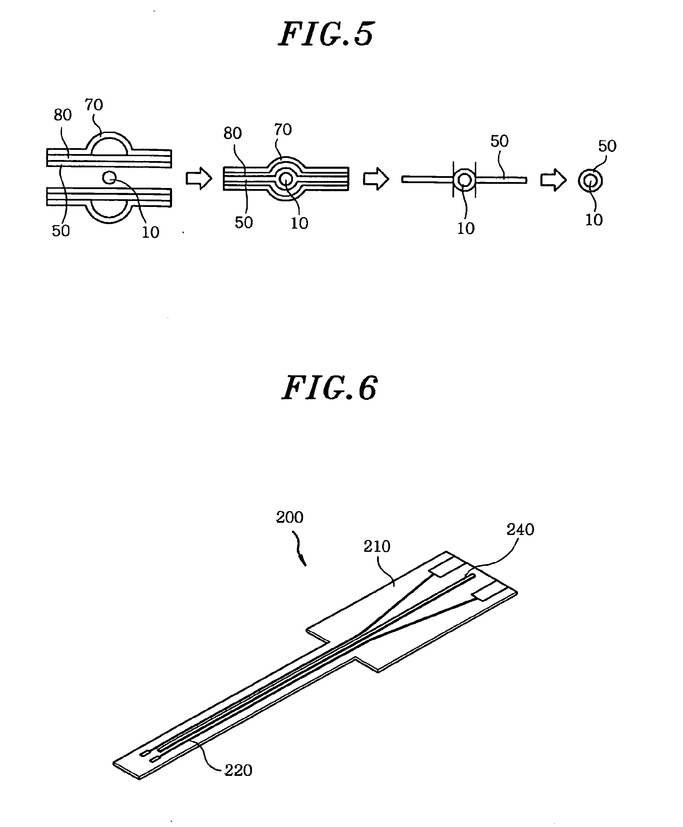

FIG. 5 illustrates another process of manufacturing the electro-optrode neural interface.

As illustrated in FIG. 5, a prepared LCP sample 50 is disposed at upper and lower positions of an optical fiber 10, and a repulsive layer 80 is disposed between the optical fiber 10 and a metal mold 70, and the resulting sample is placed in the metal mold 70.

Subsequently, the metal mold 70 is heated. Accordingly, the LCP sample 50 at the upper and lower positions of the optical fiber 10 is fused and transformed so as to surround the optical fiber 10. During the heating, the transformation of the LCP sample 50, that is, low-temperature LCP material is induced at 270°C for a predetermined period of time, after which heating is carried out under pressure so that the heating temperature is about 275 ∼ 280°C.

Subsequently, a necessary portion is cut using laser processing, and a heating process is performed, thereby forming a final electro-optrode neural interface.

FIG. 6 is a perspective view illustrating an electro-optrode neural interface according to another embodiment of the present invention. As illustrated in FIG. 6, the electro-optrode neural interface 200 according to another embodiment of the present invention includes a substrate portion 210 made of high-temperature or low-temperature LCP, electrode portions 220 formed on the upper side of the substrate portion 210 and spaced apart from each other to collect biosignals and to transfer the collected biosignals, and an optrode portion 240 formed on the upper side of the substrate portion 210 between the electrode portions 220 to form an optical electrode portion.

The electro-optrode interface 200 according to the present invention is connected to an implanted device (not shown), so that biosignals collected in vivo are transferred to the implanted device, and the implanted device performs signal processing, storage and analysis of the transferred biosignals and sends the obtained results to the outside.

The electrode portions 220 are formed by patterning a metal such as Au on the substrate portion 210. The electrode portions 220 may be formed with a multi-channel electrode, or may be formed using a MEMS process.

The optrode portion 240 is formed by patterning a patternable transparent material, that is, a photopolymer such as SU-8, and may be formed to be a desired size, enables light to pass therethrough, and may be formed using a MEMS process.

The substrate portion 210 functions to protect the electrode portions 220 and the optrode portion 240, and is made of LCP, especially low-temperature LCP.

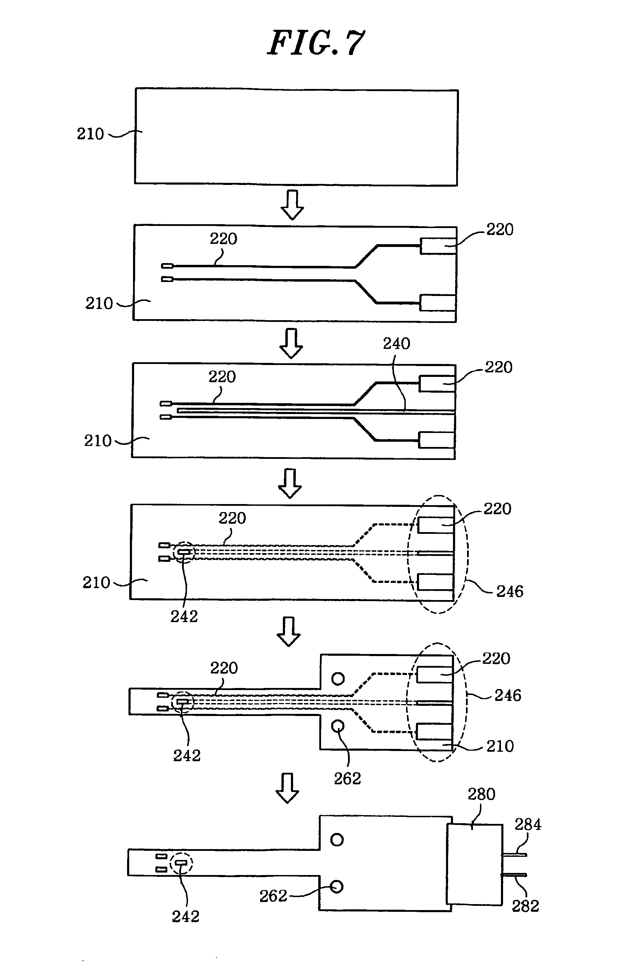

With reference to FIG. 7, the electro-optrode neural interface 200 further includes a cover portion 260 layered on the substrate portion 210 so as to protect the electrode portions 220 and the optrode portion 240.

The cover portion 260 is formed using hot pressing on the substrate portion 210, and is made of high-temperature or low-temperature LCP, especially low-temperature LCP, as in the substrate portion 210. In the case of high-temperature LCP, an adhesive layer of low-temperature LCP may be additionally required between the substrate portion 210 and the cover portion 260.

The electro-optrode neural interface according to another embodiment of the present invention is manufactured as illustrated in FIG. 7.

The upper side of a substrate portion 210 having an approximate rectangular shape is subjected to Au patterning, thus forming electrode portions 220 extended in a longitudinal direction of the substrate portion 210 and spaced apart from each other. Subsequently, the space between the electrode portions 220 is patterned with a photopolymer such as SU-8 so as not to overlap with the electrode portions 220 formed on the substrate portion 210, thus forming an optrode portion 240.

A cover portion 260 made of LCP is placed on the substrate portion 210 having the electrode portions 220 and the optrode portion 240 formed thereon and then laminated. As such, the cover portion 260 is laminated so as to provide exposure portions 242 and 246 which expose parts of the electrode portions 220 and the optrode portion 240. The exposure portion 242 is formed close to the insertion direction of the body, through which biosignals are collected. The exposure portion 246, which is located opposite to the exposure portion 242, functions to transfer the biosignals collected by the exposure portion 242 to an implanted device (not shown).

Subsequently, a part of the substrate portion 210 other than the electrode portions 220 and the optrode portion 240 formed on the substrate portion 210 laminated with the cover portion 260 is cut using a laser, thus manufacturing the electro-optrode neural interface 200 as illustrated in FIG. 6.

To connect a feed-through 280 to the manufactured electro-optrode neural interface 200, alignment holes 262 are formed in the interface, and the feed-through 280 is connected using the alignment holes 262.

The feed-through 280 includes an electrical cable 282 so that the electrode portions 220 are connected to an electrical circuit element of the implanted device, and the optrode portion 240 is connected to a measurement element of the implanted device which measures the generation of an optical signal. The feed-through is coated with an insulating material so as to prevent it from being exposed to the outside.

Although the preferred embodiments of the present invention regarding the LCP-based electro-optrode neural interface and the method of manufacturing the same according to the present invention have been disclosed for illustrative purposes, the present invention is not limited to such embodiments, and those skilled in the art will appreciate that various modifications, additions and substitutions are possible, without departing from the scope of the invention as disclosed in the accompanying claims.

## Claims

1. A liquid crystal polymer (LCP)-based electro-optrode neural interface (100) **characterised by** comprising:
an optical fiber (10), which is extended to be insertable into a body and is positioned at a core thereof so as to form an optical electrode portion; and
an LCP sample (30) surrounding the optical fiber,
wherein the LCP sample includes an adhesive sheet (20) and an LCP electrode layer surrounding the adhesive sheet and coupled to the adhesive sheet.

2. The electro-optrode neural interface of claim 1, wherein the optical fiber comprises a material which withstands a temperature of 280 ∼ 290°C.

3. The electro-optrode neural interface of claim 1, wherein the LCP electrode layer includes an electrode of an electrical stimulation site made of a multi-channel metal layer using a semiconductor process or a MEMS process,
the electro-optrode neural interface includes an interface made of the optical fiber to perform optical neural signal recording and stimulation, and
the electrical stimulation site includes a fluidic channel (42) configured to inject a drug into a region where the electrode is inserted, in addition to current/optical signal recording and stimulation.

4. A method of manufacturing an LCP-based electro-optrode neural interface, comprising:
winding an LCP sample on an optical fiber;
heating the LCP sample wound on the optical fiber using a metal mold so that the LCP sample is laminated on the optical fiber; and
cutting an unnecessary tip of the optical fiber laminated with the LCP sample, thus forming the electro-optrode neural interface.

5. The method of claim 4, wherein the heating comprises placing the LCP sample wound on the optical fiber in the metal mold and heating it while being rotated, and the metal mold and the LCP sample do not come into direct contact with each other.

6. The method of claim 4, wherein the heating is performed so that a temperature applied to the LCP sample is 275 ∼ 285°C.

7. The method of claim 4, wherein the heating comprises placing the LCP sample wound on the optical fiber in the metal mold and heating it.

8. The method of claim 4, wherein the heating comprises placing the LCP sample wound on the optical fiber in the metal mold and heating it while being rotated, and is performed in such a manner that the LCP sample is periodically tapped by the metal mold at a predetermined temporal interval.

9. The method of claim 4, wherein the heating comprises disposing the LCP sample at upper and lower positions of the optical fiber, placing the LCP sample and the optical fiber in the metal mold, and heating them.

10. An LCP-based electro-optrode neural interface (100), comprising:
a substrate portion (210) made of LCP;
electrode portions (220) formed on an upper side of the substrate portion to collect biosignals and to transfer the collected biosignals; **characterised by** comprising:
an optrode portion (240) formed on the upper side of the substrate portion so as not to overlap with the electrode portions in order to form an optical electrode portion; and
a cover portion (260) made of LCP, protecting the electrode portions and the optrode portion, and laminated on the substrate portion using hot pressing so as to expose a part of the optrode portion.

11. The electro-optrode neural interface of claim 10, wherein the electrode portions are formed by patterning a metal material on the substrate portion.

12. The electro-optrode neural interface of claim 10, wherein the optrode portion is formed by patterning a photopolymer on the substrate portion.

13. A method of manufacturing an LCP-based electro-optrode neural interface, comprising:
forming electrode portions on an upper side of a substrate portion made of LCP;
forming an optrode portion on the upper side of the substrate portion so as not to overlap with the electrode portions;
laminating a cover portion made of LCP on the substrate portion so as to expose a part of the optrode portion; and
cutting a region of the substrate portion other than the electrode portions and the optrode portion of the substrate portion laminated with the cover portion, thus manufacturing the electro-optrode neural interface.

14. The method of claim 13, further comprising:
forming an alignment hole in the electro-optrode neural interface; and
connecting a feed-through to the electro-optrode neural interface using the alignment hole.

## Patentansprüche

1. Flüssigkristalline Polymerbasierte (LCP) Elektro-optroden-Neuralschnittstelle (100), **gekennzeichnet durch**:
eine optische Faser (10), die ausgefahren in einen Körper einsetzbar ist, und in einem Kern davon angeordnet ist, um so einen optischen Elektrodenabschnitt auszubilden; und
eine LCP-Probe(30), die die optische Faser umgibt,
wobei die LCP-Probe eine Klebefolie (20) und eine LCP-Elektrodenschicht enthält, die die Klebefolie umgibt und mit der Klebefolie verbunden ist.

2. Elektrooptroden-Neuralschnittstelle nach Anspruch 1, wobei die optische Faser ein Material umfasst, das einer Temperatur von 280 - 290 °C widersteht.

3. Elektrooptroden-Neuralschnittstelle nach Anspruch 1, wobei die LCP-Elektrodenschicht eine Elektrode einer elektrischen Stimulationsstelle aufweist, die aus einer Mehrkanal-Metallschicht unter Verwendung eines Halbleiterverfahrens oder eines MEMS-Verfahrens hergestellt ist,
wobei die Elektrooptroden-Neuralschnittstelle eine aus der optischen Faser bestehende Schnittstelle aufweist, um eine optisch-neurale Signalaufzeichnung und Stimulation durchzuführen, und
wobei die elektrische Stimulationsstelle einen Fluidkanal (42) aufweist, der derart konfiguriert ist, dass zusätzlich zur Strom / optischen Signalaufzeichnung und Stimulation ein Medikament in einen Bereich injiziert wird, wo die Elektrode eingeführt ist.

4. Verfahren zur Herstellung einer LCP-basierten Elektro-optroden-Neuralschnittstelle, umfassend:
Wickeln einer LCP-Probe auf eine optische Faser;
Erhitzen der auf die optische Faser gewickelten LCP-Probe unter Verwendung einer Metallform, so dass die LCP-Probe auf die optische Faser laminiert wird; und
Abschneiden einer unnötigen Spitze der mit der LCP-Probe laminierten optischen Faser, so dass die Elektrooptroden-Neuralschnittstelle ausgebildet wird.

5. Verfahren nach Anspruch 4, wobei das Erhitzen ein Anordnen der auf die optische Faser gewickelten LCP-Probe in der Metallform und ein Erhitzen, während sie gedreht wird, umfasst, und wobei die Metallform und die LCP-Probe nicht in direkten Kontakt miteinander kommen.

6. Verfahren nach Anspruch 4, wobei das Erhitzen derart durchgeführt wird, dass eine an der LCP-Probe aufgebrachte Temperatur 275 ∼ 285 °C beträgt.

7. Verfahren nach Anspruch 4, wobei das Erhitzen ein Anordnen der auf die optische Faser gewickelten LCP-Probe in der Metallform und ein Erhitzen umfasst.

8. Verfahren nach Anspruch 4, wobei das Erhitzen ein Anordnen der auf die optische Faser gewickelten LCP-Probe in der Metallform und ein Erhitzen, während sie gedreht wird, umfasst, und derart durchgeführt wird, dass die LCP-Probe in regelmäßigen Abständen durch die Metallform in einem vorgegebenen zeitlichen Abstand angezapft wird.

9. Verfahren nach Anspruch 4, wobei das Erhitzen ein Anordnen der LCP-Probe an oberen und unteren Positionen der optischen Faser umfasst, wobei die LCP-Probe und die optische Faser in die Metallform eingelegt wird und beide erhitzt werden.

10. LCP-basierte Elektrooptroden-Neuralschnittstelle (100), mit:
einem Substratabschnitt (210) aus LCP;
Elektrodenabschnitten (220), die an einer oberen Seite des Substratabschnitts ausgebildet sind, um Biosignale zu sammeln und die gesammelten Biosignale zu übertragen;
**gekennzeichnet durch**:
einen Optrodenabschnitt (240), der auf der oberen Seite des Substratabschnitts derart ausgebildet ist, um nicht mit den Elektrodenabschnitten zu überlappen und um einen optischen Elektrodenabschnitt auszubilden; und
einen Abdeckabschnitt (260) aus LCP, der die Elektrodenabschnitte und den Optrodenabschnitt schützt, und auf dem Substratabschnitt unter Verwendung von Heißpressen laminiert ist, um so einen Teil des Optrodenabschnitts freizulegen.

11. Elektrooptroden-Neuralschnittstelle nach Anspruch 10, wobei die Elektrodenabschnitte durch Strukturieren eines Metallmaterials auf dem Substratabschnitt ausgebildet sind.

12. Elektrooptroden-Neuralschnittstelle nach Anspruch 10, wobei der Optrodenabschnitt durch Strukturieren eines Photopolymers auf dem Substratabschnitt ausgebildet ist.

13. Verfahren zur Herstellung einer LCP-basierten Elektro-optroden-Neuralschnittstelle, umfassend:
Ausbilden von Elektrodenabschnitten an einer oberen Seite eines Substratabschnitts aus LCP;
Ausbilden eines Optrodenabschnitts an der oberen Seite des Substratabschnitts, wobei er nicht mit den Elektrodenabschnitten überlappt;
Laminieren eines Abdeckabschnitts aus LCP auf dem Substratabschnitt, um so einen Teil des Optrodenabschnitts freizulegen; und
Abschneiden eines Bereichs des Substratabschnitts mit Ausnahme der Elektrodenabschnitte und des Optrodenabschnitts des mit dem Abdeckabschnitt laminierten Substratabschnittes,
wodurch die Elektrooptroden-Neuralschnittstelle hergestellt wird.

14. Verfahren nach Anspruch 13, ferner umfassend:
Ausbilden eines Ausrichtungslochs in der Elektrooptroden-Neuralschnittstelle; und
Verbinden einer Durchführung zur Elektrooptroden-Neuralschnittstelle unter Verwendung des Ausrichtungslochs.

## Revendications

1. Interface neuronale électro-optrode (100) à base de polymère à cristaux liquides (LCP), **caractérisée en ce qu'**elle comprend :
une fibre optique (10), qui est déployée pour être insérable dans un corps et est positionnée au coeur de celui-ci de manière à former une portion d'électrode optique ; et
un échantillon de LCP (30) entourant la fibre optique,
l'échantillon de LCP comportant une feuille adhésive (20) et une couche d'électrode LCP entourant la feuille adhésive et couplée à la feuille adhésive.

2. Interface neuronale électro-optrode selon la revendication 1, dans laquelle la fibre optique comprend un matériau qui supporte une température de 280 - 290 °C.

3. Interface neuronale électro-optrode selon la revendication 1, dans laquelle la couche d'électrode LCP comporte une électrode d'un site de stimulation électrique constitué d'une couche métallique multicanal utilisant un processus semi-conducteur ou un processus MEMS,
l'interface neuronale électro-optrode comportant une interface constituée de la fibre optique pour effectuer l'enregistrement et la stimulation de signaux optiques neuronaux, et
le site de stimulation électrique comportant un canal fluidique (42) configuré pour injecter un médicament dans une région où l'électrode est insérée, en plus de l'enregistrement et la stimulation de signaux électriques/optiques.

4. Procédé de fabrication d'une interface neuronale électro-optrode à base de LCP, comprenant :
l'enroulement d'un échantillon de LCP sur une fibre optique ;
le chauffage de l'échantillon de LCP enroulé sur la fibre optique à l'aide d'un moule métallique de telle sorte que l'échantillon de LCP soit laminé sur la fibre optique ; et
la découpe d'un bout inutile de la fibre optique laminée avec l'échantillon de LCP, formant ainsi l'interface neuronale électro-optrode.

5. Procédé selon la revendication 4, dans lequel le chauffage comprend le positionnement de l'échantillon de LCP enroulé sur la fibre optique dans le moule métallique et son chauffage pendant qu'on le fait tourner, et le moule métallique et l'échantillon de LCP ne viennent pas en contact direct l'un avec l'autre.

6. Procédé selon la revendication 4, dans lequel le chauffage est effectué de telle sorte qu'une température appliquée à l'échantillon de LCP est de 275 - 285 °C.

7. Procédé selon la revendication 4, dans lequel le chauffage comprend le positionnement de l'échantillon de LCP enroulé sur la fibre optique dans le moule métallique et son chauffage.

8. Procédé selon la revendication 4, dans lequel le chauffage comprend le positionnement de l'échantillon de LCP enroulé sur la fibre optique dans le moule métallique et son chauffage pendant qu'on le fait tourner, et est effectué de manière à ce que l'échantillon de LCP soit périodiquement tassé par le moule métallique à un intervalle temporel prédéterminé.

9. Procédé selon la revendication 4, dans lequel le chauffage comprend la disposition de l'échantillon de LCP à des positions supérieure et inférieure de la fibre optique, le positionnement de l'échantillon de LCP et de la fibre optique dans le moule métallique, et leur chauffage.

10. Interface neuronale électro-optrode (100) à base de LCP, comprenant :
une portion de substrat (210) constituée de LCP ;
des portions d'électrode (220) formées sur un côté supérieur de la portion de substrat pour collecter des biosignaux et pour transférer les biosignaux collectés ; **caractérisée en ce qu'**elle comprend :
une portion d'optrode (240) formée sur le côté supérieur de la portion de substrat de manière à ne pas chevaucher les portions d'électrode afin de former une portion d'électrode optique ; et
une portion de couvercle (260) constituée de LCP, protégeant les portions d'électrode et la portion d'optrode, et laminée sur la portion de substrat en utilisant un pressage à chaud de manière à exposer une partie de la portion d'optrode.

11. Interface neuronale électro-optrode selon la revendication 10, dans laquelle les portions d'électrode sont formées en structurant un matériau métallique sur la portion de substrat.

12. Interface neuronale électro-optrode selon la revendication 10, dans laquelle la portion d'optrode est formée en structurant un photopolymère sur la portion de substrat.

13. Procédé de fabrication d'une interface neuronale électro-optrode à base de LCP, comprenant :
la formation de portions d'électrode sur un côté supérieur d'une portion de substrat constituée de LCP ;
la formation d'une portion d'optrode sur le côté supérieur de la portion de substrat de manière à ne pas chevaucher les portions d'électrode ;
le laminage d'une portion de couvercle constituée de LCP sur la portion de substrat de manière à exposer une partie de la portion d'optrode ; et
la découpe d'une région de la portion de substrat en dehors des portions d'électrode et de la portion d'optrode de la portion de substrat laminée avec la portion de couvercle, fabriquant ainsi une interface neuronale électro-optrode.

14. Procédé selon la revendication 13, comprenant en outre :
la formation d'un trou d'alignement dans l'interface neuronale électro-optrode ; et
la connexion d'une traversée à l'interface neuronale électro-optrode à l'aide du trou d'alignement.
